(19)

Europäisches
Patentamt
European
Patent Office
Office européen
des brevets

(11) **EP 2 957 335 B1**

(12) **EUROPEAN PATENT SPECIFICATION**

(45) Date of publication and mention
of the grant of the patent:
**27.05.2020 Bulletin 2020/22**

(51) Int Cl.:
**B01D 61/14** *(2006.01)*        **B01D 61/18** *(2006.01)*
**B01D 63/08** *(2006.01)*        **C07K 1/34** *(2006.01)*
**B01D 65/02** *(2006.01)*

(21) Application number: **15172144.6**

(22) Date of filing: **15.06.2015**

(54) **SINGLE-PASS FILTRATION SYSTEMS AND PROCESSES**

SINGLE-PASS-FILTERSYSTEME UND VERFAHREN

SYSTÈMES ET PROCESSUS DE FILTRATION MONOPASSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2014 US 201462012792 P**

(43) Date of publication of application:
**23.12.2015 Bulletin 2015/52**

(60) Divisional application:
**20167074.2**

(73) Proprietor: **EMD Millipore Corporation
Burlington, MA 01803 (US)**

(72) Inventors:
• **Lutz, Herbert**
  **Billerica, MA 01821 (US)**
• **Parrella, Joseph**
  **Billerica, MA 01821 (US)**
• **Raghunath, Bala**
  **Billerica, MA 01821 (US)**

(74) Representative: **J A Kemp LLP
14 South Square
Gray's Inn
London WC1R 5JJ (GB)**

(56) References cited:
EP-A1- 2 119 492        EP-A1- 2 703 066
WO-A2-2007/076496      WO-A2-2007/076497
US-A- 5 147 542         US-A1- 2003 066 794
US-A1- 2003 111 402     US-A1- 2004 167 320
US-A1- 2006 051 347     US-A1- 2011 005 984
US-A1- 2011 309 018     US-B1- 6 365 395
US-B1- 8 506 802

• **JEMELLE DIZON-MASPAT ET AL: "Single pass
tangential flow filtration to debottleneck
downstream processing for therapeutic antibody
production", BIOTECHNOLOGY AND
BIOENGINEERING, vol. 109, no. 4, 1 April 2012
(2012-04-01) , pages 962-970, XP055050387,
ISSN: 0006-3592, DOI: 10.1002/bit.24377**
• **TESKE ET AL.: "Inline Ultrafiltration",
BIOTECHNOL. PROG., vol. 26, no. 4, 1 March 2010
(2010-03-01), pages 1068-1072, XP002743123,**
• **PAUL G. MIDDLEWOOD ET AL: "Extraction of
amaranth starch from an aqueous medium using
microfiltration: Membrane fouling and cleaning",
JOURNAL OF MEMBRANE SCIENCE, vol.
411-412, 12 April 2012 (2012-04-12), pages 22-29,
XP055135390, NL ISSN: 0376-7388, DOI:
10.1016/j.memsci.2012.04.008**
• **CATHERINE CASEY ET AL: "Protein
concentration with single-pass tangential flow
filtration (SPTFF)", JOURNAL OF MEMBRANE
SCIENCE, ELSEVIER BV, NL, vol. 384, no. 1, 3
September 2011 (2011-09-03), pages 82-88,
XP028319561, ISSN: 0376-7388, DOI:
10.1016/J.MEMSCI.2011.09.004 [retrieved on
2011-09-10]**
• **KWANG-HO C ET AL: "Membrane fouling
mechanisms in the membrane-coupled
anaerobic bioreactor", WATER RESEARCH,
ELSEVIER, AMSTERDAM, NL, vol. 30, no. 8, 1
August 1996 (1996-08-01) , pages 1771-1780,
XP004236319, ISSN: 0043-1354, DOI:
10.1016/0043-1354(96)00053-X**

**Description**

BACKGROUND OF THE INVENTION

Single-pass tangential flow filtration (SPTFF) processes provide several advantages over recirculating, or traditional batch TFF processes, including simplicity, ease of use, and a smaller footprint, while avoiding some of the undesirable effects associated with recirculation, such as lower conversion.

SPTFF processes are typically performed at lower feed flow rates compared to traditional batch processes to facilitate conversion by increasing residence time within the module. The cassettes used in SPTFF processes are often processed in series, rather than in parallel, to improve conversion by increasing mass transfer through operation at higher feed flow rates. Although processing the cassettes in series can improve SPTFF performance and product recovery, parallel processing is often preferred for flushing SPTFF assemblies with liquids to remove preservative or storage solution, measure permeability, equilibrate the membranes, clean the membranes, or prepare the membranes for storage, particularly because serial processing requires additional time and material (e.g., water, buffer, cleaning solutions, storage solutions), which increases the overall cost of operation compared to parallel processing.

Since serial staging is desirable for product (e.g., protein) processing and parallel staging is desirable for flushing and cleaning SPTFF assemblies, there is a present need for SPTFF systems that can readily transition from processing cassettes in series to processing cassettes in parallel, and *vice versa*, while maintaining a sanitary processing environment.

In addition, current SPTFF systems suffer from a number of drawbacks, including lack of disposability for single-use operation, insufficient scalability (e.g., for linear scale-up or scale-down) and inadequate cleaning and product recovery methods.

Accordingly, there is a need for improved SPTFF assemblies that are scalable and/or disposable, as well as a need for more effective methods for cleaning and recovering target products (e.g., proteins) from the filtration membranes in TFF cassettes.

US 2003/0111402 discloses an apparatus for the separation of fluid mixtures into a permeate and a retentate comprises separation modules each having fluid containment chambers stacked therein. Each fluid containment chamber is defined by a pair of selectively permeable membranes having their active surfaces facing inwardly toward one another, with a gasket sandwiched therebetween in sealing engagement with each membrane. A channelled support plate is positioned adjacent to the outside surface of each membrane. The channels in the support plates fluidly communicate with a housing, thereby allowing negative pressure from the housing to be brought into direct contact with the outside surface of each selectively perme-able membrane. The fluid mixture is pumped into each fluid containment chamber at one end and moves across the active surfaces of the membranes toward a retentate receiver positioned at the opposite end. In doing so, permeate is drawn out through both membranes into the channels and the housing. The housing has a top panel which is adjustable to ensure ongoing sealing engagement of the gaskets with the membranes.

WO 2007/076496 discloses a system, method and device for bio-processing a feed stream and providing a constant output by operating a continuous single-pass tangential-flow process. The single-pass process provides high conversion concentration while operating at relatively low feed flow rates, and the process can also be used to provide constant output diafiltration

SUMMARY OF THE INVENTION

The present invention provides, in one embodiment, a single pass tangential flow filtration (SPTFF) assembly according to claim 1. In a particular embodiment, the valves in the SPTFF assembly are sanitary valves.

BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

FIG. 1 is a diagram depicting a standard TFF system with three processing units that are operated in parallel.
FIG. 2 is a diagram depicting a TFF system of the invention having three processing units that can be operated in parallel or in series, showing open valves for operation in parallel.
FIG. 3 is a diagram depicting a TFF system of the invention having three processing units that can be operated in parallel or in series, showing closed valves for operation in series.
FIG. 4 is a diagram of a spool piece for a hybrid SPTFF assembly of the invention.

FIG. 5 is a diagram depicting a disposable SPTFF assembly having two processing units.

FIG. 6 is a diagram depicting a scalable SPTFF assembly having two processing units.

FIG. 7 is a diagram depicting a TFF system having three processing units and showing seals between adjacent processing units for operation in series.

FIG. 8 shows Normalized Buffer Permeability (NBP) recovery after static soak cleaning.

FIG. 9 shows the process flux after re-use with static soak cleaning.

## DETAILED DESCRIPTION OF THE INVENTION

*Definitions*

Unless defined otherwise, all technical and scientific terms used herein have the same meaning as commonly understood by one of ordinary skill in the art to which this invention pertains.

"SPTFF assembly," "SPTFF system" and "SPTFF apparatus" are used interchangeably herein to refer to a TFF system that is configured for operation in a single-pass TFF mode.

"Single-pass TFF mode" refers to operating conditions for a TFF system/assembly under which all or a portion of the retentate is not recirculated through the system.

The terms "feed," "feed sample" and "feed stream" refer to the solution being fed to the filtration module for separation.

The term "separation" generally refers to the act of separating the feed sample into two streams, a permeate stream and a retentate stream.

The terms "permeate" and "permeate stream" refer to that portion of the feed that has permeated through the membrane.

The terms "retentate" and "retentate stream" refer to the portion of the solution that has been retained by the membrane, and the retentate is the stream enriched in a retained species.

"Feed line" or "feed channel" refers to a conduit for conveying a feed from a feed source (e.g., a feed container) to one or more processing units in a filtration assembly (e.g., an SPTFF assembly).

"Retentate line" or "retentate channel" refers to a conduit in a filtration assembly for a retentate or retentate stream.

"Permeate line" or "permeate channel" refers to a conduit in a filtration assembly for a permeate or permeate stream.

The expression "flow path" refers to a channel comprising a filtration membrane (e.g., ultrafiltration membrane, micro filtration membrane) through which the solution being filtered passes in a tangential flow mode. The flow path can have any topology which supports tangential flow (e.g., straight, coiled, arranged in zigzag fashion). A flow path can be open, as in an example of channels formed by hollow fiber membranes, or have one or more flow obstructions, as in the case, for example, of rectangular channels formed by flat-sheet membranes spaced apart by woven or non-woven spacers.

A "processing unit" or "unit" refers to a cassette holder comprising one or more cassettes.

A "cassette holder" refers to a container for one or more cassettes. Typically, when a cassette holder contains more than one cassette, the cassettes are configured for parallel processing, although, in some embodiments, the cassettes can be configured for serial processing.

A "cassette" refers to a cartridge or plate-and-frame structure comprising a filtration membrane (e.g., an ultrafiltration membrane, a micro filtration membrane) suitable for TFF processes.

"Filtration membrane" refers to a selectively permeable membrane for separating a feed into a permeate stream and a retentate stream using a TFF process. Filtration membranes include, but are not limited to, ultrafiltration (UF) membranes, microfiltration (MF) membranes, reverse osmosis (RO) membranes and nanofiltration (NOF) membranes.

The terms "ultrafiltration membrane" and "UF membrane" are used herein to refer to a membrane that has pore sizes in the range of between about 1 nanometer to about 100 nanometers.

The term "microfiltration membranes" and "MF membranes" are used herein to refer to membranes that have pore sizes in the range between about 0.1 micrometers to about 10 micrometers.

The term "plurality," when used herein to describe processing units, refers to two or more (e.g., two, three, four, five, etc.) processing units.

"Fluidly connected" refers to a plurality of processing units that are connected to one another by one or more conduits for a liquid, such as, for example, a feed channel, retentate channel and/or permeate channel.

"Product" refers to a target compound in a feed. Typically, a product will be a biomolecule (e.g., protein) of interest, such as a monoclonal antibody (mAb).

"Processing" refers to the act of filtering (e.g., by SPTFF) a feed containing a product of interest and subsequently recovering the product in a concentrated form. The concentrated product can be recovered from the filtration system (e.g., a SPTFF assembly) in either the retentate stream or permeate stream depending on the product's size and the pore size of the filtration membrane.

The expressions "parallel processing", "processing in parallel", "parallel operation" and "operation in parallel" refer to processing a product in a TFF assembly (e.g., SPTFF assembly) that contains a plurality of processing units that are fluidly connected by distributing the feed directly from the feed channel to each of the processing units in the assembly.

The expressions "serial processing", "processing in series", "serial operation" and "operation in series" refer to processing a product in a TFF assembly (e.g., SPTFF assembly) that contains a plurality of processing units that are fluidly connected by distributing the feed directly from the feed channel to only the first processing unit in the assembly. In serial processing, each of the other, subsequent processing units in the assembly receives its feed from the retentate line of the preceding processing unit (e.g., the retentate from a first processing unit serves as the feed for a second, adjacent processing unit). The expressions "conversion," "single-pass conversion," and "conversion per pass" are used herein to denote the fraction of the feed volume that permeates through the membrane in a single pass through the flow channels, expressed as a percentage of the feed stream volume.

The term "residence time" refers to holdup volume divided by flow rate.

*Single-Pass Tangential Flow Filtration (TFF) Systems*

Tangential flow filtration (TFF) is a separation process that uses membranes to separate components in a liquid solution or suspension on the basis of size, molecular weight or other differences. In traditional TFF processes, the fluid is pumped tangentially along the membrane surface and particles or molecules which are too large to pass through the membrane are rejected and returned to a process tank for additional passes across the membrane (i.e., recirculation) until the process fluid is sufficiently clarified, concentrated or purified. The cross-flow nature of TFF minimizes membrane fouling, thus permitting high volume processing per batch.

Traditional batch-fed recirculation TFF processes, however, are limited due to the size and minimum working volume of existing TFF systems. Single-Pass TFF (SPTFF) allows direct flow-through concentration of a product (e.g., protein) in the absence of recirculation, which reduces overall system size through elimination of mechanical components and permits continuous operation at high conversion levels. While existing SPTFF systems and processes provide several advantages over traditional recirculating TFF systems and processes, there is a need for improved SPTFF systems and processes that provide users with added operating flexibility, reduced processing time and buffer requirements, and increased product yield and recovery.

In general, the SPTFF systems/assemblies described herein (also referred to as SPTFF systems/assemblies of the invention) can be assembled and operated using standard, existing TFF system components that are well known and are commercially available. Standard TFF system components that are commercially available generally include, for example, TFF cassettes comprising filtration membranes, cassette holders, conduits (e.g., tubing, piping) for feed, retentate and permeate, a housing or enclosure, valves, gaskets, a pump module (e.g., pump module comprising a pump housing, diaphragm and check valve) one or more reservoirs (e.g., bioprocess containers) and a pressure gauge.

Exemplary cassettes (e.g., TFF cassettes) that are suitable for use in the SPTFF systems of the invention include, but are not limited to, TFF cassettes supplied by EMD Millipore Corporation (Billerica, MA), such as, for example, Pellicon® cassettes with Biomax™ membrane or Ultracel™ membrane (e.g., Pellicon® XL 50 cassettes, Pellicon® 2 cassettes, Pellicon® 2 Mini cassettes, Pellicon® 2 Maxi cassettes, Pellicon® 3 cassettes), Prostak™ microfiltration modules, and Prep/Scale® TFF cartridges). Additional TFF cassettes capable of being used in SPTFF systems include, e.g., T-series cassettes with Delta membrane for Cadence™ SPTFF modules (Pall Corporation, Port Washington, NY), Kvick™ Flow cassettes (GE Healthcare Bio-Sciences, Piscataway, NJ) and Hydrosart® cassettes (Sartorius AG, Bohemia, NY).

Cassette holders that are suitable for use in SPTFF assemblies include, for example, Pellicon® cassette holders (EMD Millipore Corporation, Billerica, MA) such as, for example, Pellicon® 2 miniholders, acrylic Pellicon® holders, stainless steel Pellicon® holders, process scale Pellicon® holders. Other suitable cassette holders include, but are not limited to, Centramate™ TFF membrane cassette holders, Centrasette™ TFF membrane cassette holders, Maximate™ TFF membrane cassette holders and Maxisette™ TFF membrane cassette holders (Pall Corporation, Port Washington, NY). In some embodiments, existing cassette holders (e.g., Pellicon® cassette holders (EMD Millipore Corporation)) can be modified to function in the hybrid, disposable, scalable and/or adapatable SPTFF assemblies described herein.

In various embodiments, the SPTFF system components can be disposable. Exemplary disposable components for SPTFF assemblies include, but are not limited to, components of Flexware® assemblies for Mobius® FlexReady Solution for TFF (EMD Millipore Corporation, Billerica, MA). Other disposable components for SPTFF assemblies include, for example, components of Allegro™ TFF assemblies (Pall Corporation, Port Washington, NY).

*Hybrid SPTFF Systems*

For biotechnology applications, ultrafiltration (UF) cassettes are typically run in a batch process where the retentate is returned to a retentate tank and circulated through the cassette multiple times. Single-pass TFF (SPTFF) allows continuous processing but can require more membrane area. It has advantages in niche applications such as de-watering (concentration) to debottleneck tanks or increase column utilization, achieve higher final concentrations, and buffer condition fluids before columns. For achieving higher final concentrations, SPTFF avoids constraints on batch systems such as system footprint, a minimum working volume or dilution during product recovery.

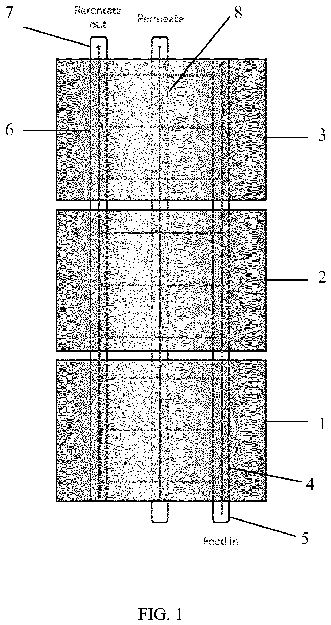

TFF systems operated in parallel typically have processing units (e.g., cassette holders containing TFF cassettes) that are stacked vertically. The vertical stack of process holders typically shares a feed line, a retentate line, and a permeate line in common for parallel operation (see FIG. 1). However, optimized implementation of an SPTFF assembly utilizes processing units that are operated in series such that the retentate from a processing unit serves as feed for the subsequent adjacent processing unit.

Since serial operation is advantageous for product (e.g., protein) processing and recovery, and parallel operation is desirable for flushing and cleaning TFF assemblies, the present invention contemplates a TFF system (e.g., a SPTFF system) that is configured for processing in series and in parallel, referred to herein as a "hybrid system" or "hybrid assembly." The hybrid system can be operated in parallel for particular processes (e.g., flushing, equilibration, cleaning, preparation for storage, measurement of NWP), or in series for product processing and recovery.

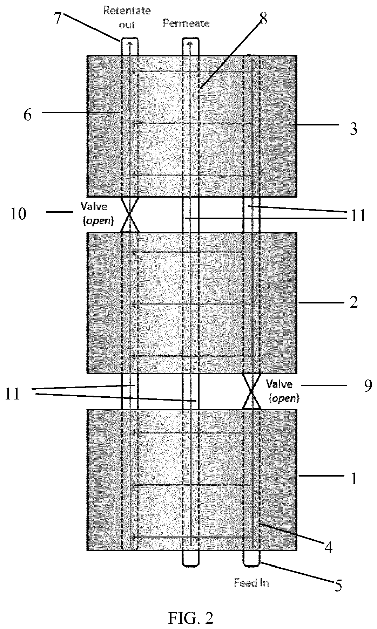

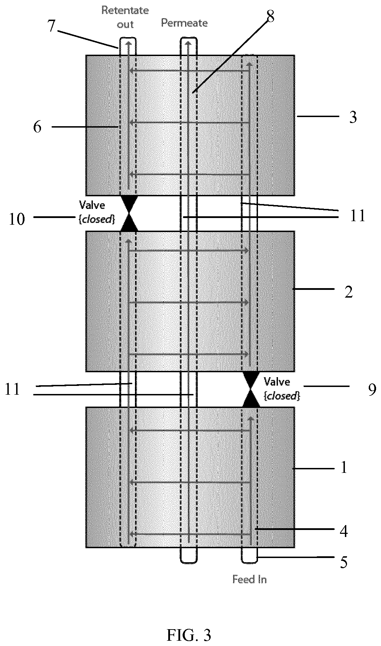

Accordingly, in one embodiment, the invention provides a single-pass tangential flow filtration (SPTFF) assembly comprising a plurality of processing units that are fluidly connected, wherein each processing unit comprises a cassette holder containing at least one TFF cassette, and wherein the processing units in the assembly are configured for processing in parallel and/or in series; a first channel connected to a feed inlet extending through the plurality of processing units; a second channel connected to a retentate outlet extending through the plurality of processing units; a permeate channel extending through the plurality of processing units; and one or more valves located on either the first channel or the second channel between two adjacent processing units. According to this aspect of the invention, the valves alternate between the first channel and second channel for consecutive pairs of adjacent processing units, such that the plurality of processing units are processed in parallel when all of the valves are open (FIG. 2), or in series when all of the valves are closed (FIG. 3).

The valves are built into the cassette holder of each processing unit. Preferably, the valves are sanitary valves. As used herein, a "sanitary valve" is a valve that can maintain a sterile connection regardless of whether the valve is open or closed. Typically, a sanitary valve will be compatible, non-toxic, sanitizeable and non-shedding.

A hybrid SPTFF system of the invention can have two or more stages. In one embodiment, a hybrid SPTFF assembly has only two processing units. When there are only two processing units, a single valve on the first channel between the two processing units can be used to toggle between serial and parallel processing. For example, the two processing units are operated in parallel when the valve on the first channel is open, and operated in series when the valve is closed.

Preferably, hybrid SPTFF assemblies of the invention include three or more (e.g., four, five, six, seven, eight) processing units. The processing units can be stacked (e.g., stacked vertically) to form a stacked assembly.

When there are three or more processing units, the valves on the first channel alternate with valves on the second channel between consecutive pairs of adjacent processing units (e.g., a series of three processing units, wherein the first and second processing units in the series constitutes a first pair of adjacent processing units, and the second and third processing units in the series constitutes a second pair of adjacent processing units, wherein the first and second pairs of processing units are ordered consecutively in the series). For example, a hybrid SPTFF assembly having three processing units preferably has a valve positioned on the first channel between the first and second processing units in the series, while a second valve is positioned on the second channel between the second and third processing units in the series (see, e.g., FIGS. 2 and 3). In another example, a hybrid SPTFF assembly having a series of five processing units has valves on the first channel between the first and second processing units and between the third and fourth processing units in the series, while valves are positioned on the second channel between the second and third processing units and the fourth and fifth processing units in the series.

FIG. 1 depicts a valve-less SPTFF assembly having three processing units **1, 2, 3** that are fluidly connected, a first channel **4** extending from a feed inlet **5** through each of the processing units **1, 2, 3,** a second channel **6** that extends through the processing units **1, 2, 3** and is connected to a retentate outlet **7,** and a permeate channel **8** extending through the processing units **1, 2, 3.** Vertical arrows show the direction of the flowpaths for the feed, retentate and permeate, while horizontal arrows show the direction of the flowpath through the processing units. In the absence of valves on the first and second channels, the feed is distributed in a parallel fashion to all three of the processing units through the first channel, while retentate from each of the processing units flows through the second channel and out of the assembly through the retentate outlet.

FIGS. 2 and 3 depict a similar SPTFF assembly having three processing units **1, 2,** and **3** that are fluidly connected, except that the assemblies in FIGS. 2 and 3 each contain a valve **9** on the first channel between adjacent processing units **1, 2,** and a valve **10** on the second channel between a subsequent consecutive pair of adjacent processing units **2, 3.** Valve-less spool pieces **11** are located on the second channel **6** and permeate channel **8** between processing units **1** and **2,** and on the first channel **4** and permeate channel **8** between processing units **2** and **3.** In FIG. 2, the valve **9** on the first channel and valve **10** on the second channel are both open to allow for parallel processing of the processing units **1, 2, 3** (e.g., for flushing or cleaning the assembly). In contrast, the valve **9** on the first channel and valve **10** on the second channel in FIG. 3 are both closed to allow for serial processing of the three processing units **1, 2, 3** (e.g., for product processing and recovery).

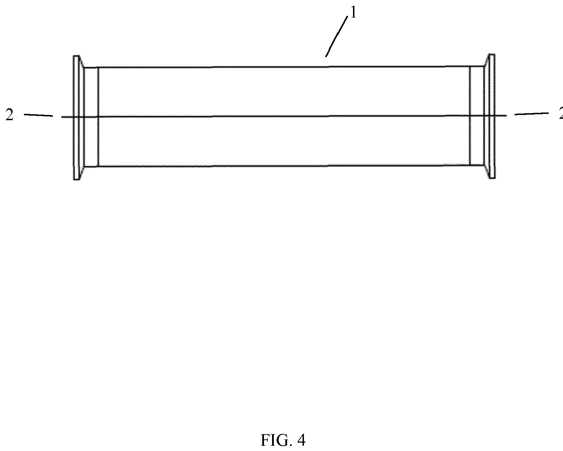

In some embodiments, each valve in the hybrid assembly has a corresponding spool piece located on a different channel between the same processing units. In general, the spool piece is an open conduit with sanitary ends that connects the retentate channel from one process holder to the feed channel of the following process holder, or connects the permeates channels of adjacent process holders. Preferably, the length of the spool piece is chosen to match that of the sanitary valve so the assembly has a balanced height. An exemplary spool piece for a hybrid SPTFF assembly described herein is depicted in FIG. 4. The spool piece in FIG. 4 includes an open cylindrical body **1** capable of acting as a fluid conduit with flanges **2** located at both ends. In some embodiments, the hybrid assembly can have a valve on the first channel and a spool piece on the second channel between the first and second processing units in a series of two processing units. Alternatively, or additionally, the assembly can have a spool piece on the first channel and a valve on the second channel between the same two adjacent processing units. For example, in a hybrid SPTFF assembly having three processing units, there can be a valve on the first channel and a spool piece on the second channel between the first and second processing units, and a valve on the second channel and a spool piece on the first channel between the second and third processing units (see, e.g., FIGS. 2 and 3).

Preferably, the hybrid SPTFF assembly is in a container (e.g., housing) that shields the assembly and its components from the external environment. For example, the assembly (e.g., stacked assembly) can be inserted into a rigid container that applies a compressive force to seal the assembly from the external environment. The disposable container (e.g., a rigid holder) can include tubing (e.g., disposable tubing) at each end for making connections (e.g., aseptic connections) to feed and product containers (e.g., vessels, bags), respectively, such that an operator of the hybrid assembly can toggle between serial and parallel operation without opening the system to the outside environment and/or removing devices from the holders.

*Disposable SPTFF Systems*

Traditionally, a TFF process involves time consuming and costly validation of both TFF system and cassette cleaning. The use of disposable components would eliminate the need for membrane and system cleaning as well as mitigate risk of product carryover from one batch to the next. These assemblies are a complete, self-contained TFF flow path consisting of a process container, tubing, connectors, and disposable cassette manifolds that eliminate fluid contact from any reusable components. Using disposable components in TFF processing provides many benefits without sacrificing robust manufacturing processes.

SPTFF assemblies have the advantages of simplicity (e.g., no tank, no retentate recycle, no control skid) and ease of use over conventional batch TFF apparatuses. The simplicity and ease-of-use features of SPTFF assemblies can be improved by making single-use, disposable SPTFF assemblies that can facilitate plug-and-play installation and easy disassembly of flowpath components, and require no cleaning or sanitization between operations.

Thus, there is disclosed a disposable single-pass tangential flow filtration (SPTFF) assembly, comprising one or more disposable components, wherein fluid contact surfaces on the disposable components are contained (e.g., enclosed) in a disposable container (e.g., a sealed disposable container). Preferably, the disposable container (e.g., a rigid holder) includes tubing (e.g., disposable tubing) at each end for making connections (e.g., aseptic connections) to feed and product containers (e.g., vessels, bags), respectively.

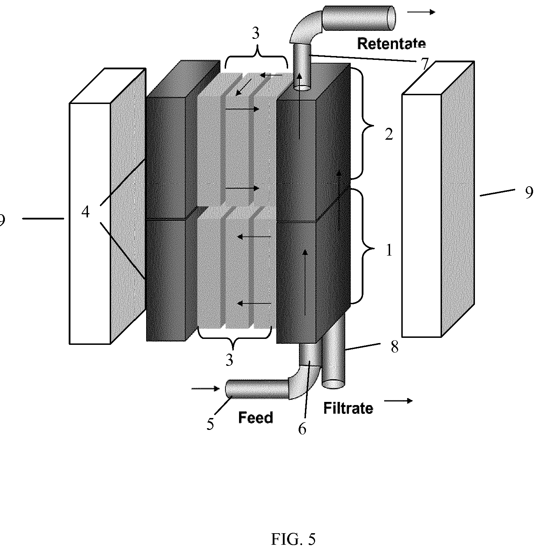

Typically, a disposable SPTFF assembly of the invention includes a disposable feed line, a disposable TFF cassette, a disposable TFF cassette holder, a disposable retentate line, and a disposable retentate valve, or a combination thereof. The disposable components and container are made from disposable materials (e.g., plastic, rubber, metal). Preferably, the disposable components and container are made from plastic. An exemplary disposable SPTFF assembly of the invention is depicted in FIG. 5. The disposable SPTFF assembly in FIG. 5 has two processing units **1, 2.** Horizontal arrows indicate the flow direction of the feed liquid through the disposable TFF cassettes **3** in each processing unit while vertical arrows show the direction of the flowpath between the processing units **1, 2.** Disposable liners **4** are positioned adjacent to the disposable TFF cassettes **3.** Other components shown include a disposable feed inlet **5** connected to a disposable feed channel **6,** a disposable retentate outlet **7,** a disposable permeate outlet **8** and disposable holder plates **9.**

Disposable SPTFF assembly components are well known and are available commercially. Exemplary disposable components for SPTFF assemblies include, but are not limited to, components of Flexware® assemblies for Mobius® FlexReady Solution for TFF (EMD Millipore Corporation, Billerica, MA). Other disposable components for SPTFF assemblies include, for example, components of Allegro™ TFF assemblies (Pall Corporation, Port Washington, NY).

Typically, the disposable feed and retentate lines are made from disposable tubing. In one embodiment, the disposable retentate line further comprises a T line (e.g., a disposable T line) for in-line buffer addition (e.g., for a diafiltration-type mode of operation).

In one embodiment, the disposable SPTFF assembly includes a disposable retentate valve that is a pinch valve (e.g., diaphragm valve) for disposable tubing. The pinch valve can be disposable or removable/re-useable. Preferably, the valve is low shear and sanitary (e.g., compatible, non-toxic, sanitizeable, non-shedding).

In certain embodiments, the disposable SPTFF assembly further comprises a disposable pressure sensor. In other embodiments, the disposable SPTFF assembly comprises a disposable diaphragm for a pressure sensor, through which pressure is monitored with a re-usable sensor.

In a particular embodiment, the disposable SPTFF assembly comprises a plurality of processing units that are fluidly connected, wherein each processing unit comprises at least one TFF cassette in a cassette holder. In a further embodiment, the plurality of processing units are stacked (e.g., stacked vertically) to produce a stacked assembly. For example, the processing units can be stacked for use with diverter plates (e.g., separator plates for Pellicon® 2 Mini Holders (EMD Millipore, Billerica, MA)). The stacked assembly can be inserted into a rigid container that would apply a compressive force to seal the assembly from the external environment and prevent bypass flow from the membrane upstream (feed/retentate) side to the membrane downstream (permeate) side, thereby producing a sealed disposable container.

The disposable SPTFF assembly can also comprise one or more disposable end plates (e.g., disposable retentate end plates). For example, larger-sized disposable SPTFF assemblies (e.g., assemblies having a filtration area greater than 5m$^2$) can include a disposable feed end plate and a disposable retentate end plate that would bypass the process holder but still allow the compressive force in the holder to work and seal the cassettes.

In some embodiments, one or more of the disposable components in the disposable SPTFF assembly can be replaceable (e.g., capable of plug-and-play installation and disassembly).

Preferably, the disposable SPTFF assembly is sanitized (e.g., sterilized) prior to use in a SPTFF process. Suitable methods for sanitizing SPTFF assemblies can readily be determined by persons of ordinary skill in the art based on compatibility of the sanitization method with the material(s) in the assembly. Exemplary sanitization methods include treatment of the assembly with a suitable sanitization agent such as, for example, gamma radiation, ethylene oxide (ETO), bleach (e.g., Clorox), chlorine (e.g., NaOCl), peroxide, acid (e.g., peracetic acid), base (e.g., NaOH), formaldehyde (e.g., formaline solution) or heat, or an appropriate combination thereof.

Installation of a disposable SPTFF assembly typically involves establishing aseptic connections between the disposable SPTFF assembly and feed and product containers at opposing ends. For example, the disposable tubing of the feed line for the SPTFF assembly can be connected to a feed container and the disposable tubing of the retentate line for the SPTFF assembly can be connected to a product container.

Accordingly, also disclosed is a method of installing a disposable single-pass tangential flow filtration (SPTFF) assembly, comprising establishing a first aseptic connection between a feed line of a disposable SPTFF assembly and a feed container, and establishing a second aseptic connection between a retentate line of the disposable SPTFF assembly and a product container.

*Scalable SPTFF Assemblies and Processes*

Small scale SPTFF systems are typically used for testing and analysis while large scale SPTFF systems are commonly used in manufacturing operations. There is an existing need for scalable systems that can transition between small scale testing and large scale manufacturing. Such scalable systems preferably can be operated using existing, commercially available components without the need for system redesign.

Linear scaling represents one means of achieving the scaling-up or scaling-down of SPTFF assemblies while ensuring that the performance between the larger and smaller scales is comparable (see, for example, van Reis, et al., "Linear Scale Ultrafiltration," Biotechnology and Bioengineering 55(5): 737-746 (1997)). As used herein, "scaling-up" refers to increasing the processing capacity of a SPTFF assembly while maintaining both the residence time of the sample and the length of the flowpath, and "scaling-down" refers to decreasing the processing capacity of a SPTFF assembly while maintaining both the residence time of the sample and the length of the flowpath (e.g., for process development and trouble-shooting purposes).

Thus, also disclosed is a method of increasing the processing capacity of a single-pass tangential flow filtration (SPTFF) assembly, comprising the steps of increasing the filtration membrane area in a SPTFF assembly; and increasing the volume of feed solution that is being delivered to the SPTFF assembly by an amount that is proportional to the increase in filtration membrane area (e.g., by increasing the volumetric flow). The volumetric flow can be increased, for example, using a pump or vacuum to apply pressure, or using gravity. According to this aspect of the invention, both the residence time of the feed solution in the SPTFF assembly and the length of the flowpath are maintained.

The filtration membrane area can be increased while maintaining the length of the flowpath by replacing one or more cassettes in the assembly with an equal number of cassettes having a greater filtration membrane area, by adding additional cassettes to the assembly, or a combination of the two. For example, one or more cassettes (e.g., minicassettes) having an effective filtration area of, e.g., 0.05 m$^2$ or 0.1 m$^2$ in a small scale SPTFF assembly can be replaced by an equal number of larger TFF cassettes having an effective filtration area of, e.g., 1.0 m$^2$ or 2.5 m$^2$ to increase the overall filtration area in the assembly. Typically, up to about 10 m$^2$ of effective filtration area can be attained on each side of the manifold segment in a processing unit. The effective filtration area can be added in parallel or in series.

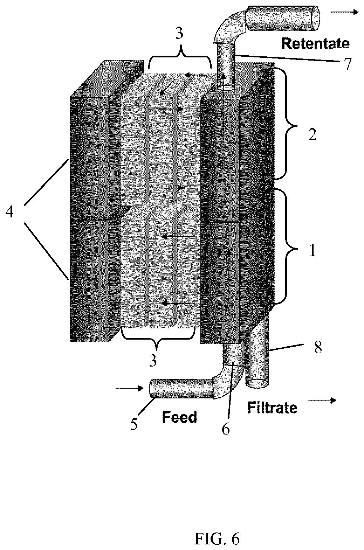

FIG. 6 depicts a scalable SPTFF assembly having two processing units **1, 2,** each processing unit having three TFF cassettes **3**. Scaling of the assembly can be achieved without altering the flowpath length through processing units **1, 2** by replacing one or more of the TFF cassettes **3** in each processing unit with an equal number of TFF cassettes having a greater (for scaling up) or lesser (for scaling down) filtration membrane area. Alternatively, scaling of the assembly can be achieved without altering the flowpath length by adding or subtracting TFF cassettes (e.g., TFF cassettes having the same effective filtration area) to or from each processing unit, such that each processing unit will contain from 1 to about 10 TFF cassettes.

*SPTFF Assemblies Comprising Mechanical Seals for Serial Processing (Not according to the invention)*

Serial processing of a SPTFF system can also be achieved using a mechanical seal (e.g., a rigid plate, a flexible gasket) that is positioned on the channel that serves as the feed line between two adjacent processing units that are processed in parallel. The mechanical seal can prevent feed flow from distributing to adjacent processing units, enabling the retentate from the first processing unit to flow to the subsequent adjacent processing unit as its feed. As a result, the two processing units are processed in series.

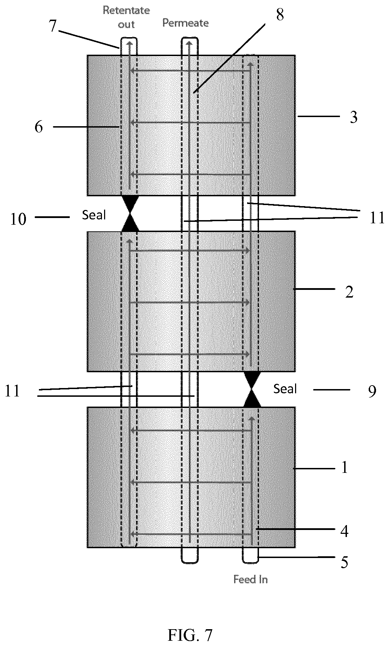

FIG. 7 depicts a SPTFF assembly having three processing units **1, 2,** and **3** that are fluidly connected. A first seal **9** is positioned on the first channel **4** that is connected to the feed inlet **5** between adjacent processing units **1, 2,** and a second seal **10** is positioned on the second channel **6** between a subsequent consecutive pair of adjacent processing units **2, 3** to allow for serial processing of the three processing units **1, 2, 3** (e.g., for product processing and recovery). Spool pieces **11** are positioned on the second channel **6** and permeate channel **8** between processing units **1** and **2,** and on the first channel **4** and permeate channel **8** between processing units **2** and **3.** In the absence of the seals, filtrate (permeate) can pass directly from one processing unit to the other. In contrast, when the seals are present, feed is prevented from flowing directly from the first processing unit **1** to the second processing unit **2.** Accordingly, retentate from the first processing unit **1** serves as the feed for the second processing unit **2,** thereby ensuring serial processing.

Accordingly, also disclosed is a method of adding a processing unit to a single-pass tangential flow filtration (SPTFF) assembly. The method comprises establishing a fluid connection between a first processing unit in the SPTFF assembly (e.g., a processing unit in the assembly that is connected to a retentate outlet) and a second processing unit that is to be added to the SPTFF assembly (e.g., an isolated processing unit or a first processing unit in a series of two or more processing units to be added to the assembly). The first and second processing units each contain a cassette holder containing at least one TFF cassette and are fluidly connected to each other by one or more fluid channels. Preferably, the first and second processing units are fluidly connected by a first channel connected to a feed inlet, a second channel connected to a retentate outlet and a permeate channel.

The method further involves the step of positioning a mechanical seal on the first channel between the first and second processing units, thereby preventing feed in the first channel in the first processing unit from flowing directly into the second processing unit and ensuring that the first and second processing units are processed in series (FIG. 7). For example, the mechanical seal can be positioned (e.g., inserted, included) between the flanges of two connected cylinders forming part of the feed channel of a SPTFF assembly between two adjacent processing units.

Suitable mechanical seals for use in a SPTFF assembly are well known. The mechanical seal can be a gasket, such as, for example, a gasket that closes off an opening or a gasket having a length sufficient to closes off any dead volume between the opening and a first passage in a manifold. Preferably, the gasket is flexible and sanitary (e.g., a gasket that is non-shedding, cleanable, sanitizable, and has low extractables). The gasket can include an elastomeric material or metal (e.g., a metal foil). An exemplary gasket for a SPTFF assembly is part# A84MP-G from Newman Gasket Co., Lebanon, OH.

*Adaptable Modular Single-Pass Tangential Flow Filtration (SPTFF) Systems*

In other embodiments, the invention relates to an adaptable modular single-pass tangential flow filtration (SPTFF) assembly. Typically, the adaptable modular assembly comprises at least one processing unit that comprises a cassette holder containing at least one TFF cassette; a first channel connected to a feed inlet; a second channel connected to a retentate outlet; a permeate channel; and one or more valves, between adjacent processing units in the assembly, thereby ensuring that the processing units in the assembly can be processed in series.

As used herein, "adaptable" means that the processing capacity of a modular assembly can be increased or decreased to achieve desired performance parameters, such as, but not limited to, a desired level of cleanliness of the assembly, enhanced target processing, and reduction of batch variability. The processing capacity of the adaptable assembly can be increased or decreased by, for example, adding or removing one or more processing units to or from the assembly. Alternatively, or in addition, the processing capacity of the adaptable assembly can be increased or decreased by adding or subtracting TFF cassettes from one or more processing units in the assembly, and/or by replacing one or more TFF cassettes in one or more processing units in the assembly with an equal number of TFF cassettes having a different membrane filtration area.

"Modular" refers to an adaptable assembly comprising one or more recurring components that can be added to, or removed from, the assembly to alter the processing capacity. Such recurring components include, for example, processing units and TFF cassettes.

In accordance with the invention, the at least one processing unit in the assembly can be fluidly connected to one or more additional processing units (e.g., by establishing an aseptic connection), as described herein. For example, about 1, 2, 3, 4, 5, 6, 7, etc., processing units can be added to an adaptable modular assembly already containing 1, 2, or 3 processing units.

In some embodiments, the processing unit can also accommodate one or more additional TFF cassettes. For example, about 1, 2, 3, 4, 5, 6, 7, etc., TFF cassettes can be added to a processing unit containing 1, 2, or 3 TFF cassettes. In one embodiment, the processing unit can accommodate up to about 10 TFF cassettes. The TFF cassettes being added can have the same, or a different, filtration membrane area as the TFF cassette(s) already present in the processing unit.

In further embodiments, the at least one TFF cassette in each processing unit can be replaced with one or more TFF cassettes having a different filtration membrane area. For example, if the TFF cassette in a processing unit has an effective filtration area of 0.1 $m^2$, it can be replaced with a larger TFF cassettes having an effective filtration area of, e.g., 2.5 $m^2$. The effective filtration area can be added in parallel or in series.

The adaptable modular assembly comprises one or more valves between adjacent processing units, as described herein above for hybrid SPTFF systems of the invention. The presence of the valves ensures that the processing units in the adaptable assembly can be processed in series when the valves are closed, which can be advantageous for sample processing, or in parallel when the valves are open, which is useful for cleaning the assembly.

In an alternative arrangement not according to the invention, the adaptable modular assembly comprises one or more seals between adjacent processing units, as described herein above for SPTFF assemblies comprising mechanical seals. The presence of the seals ensures that the processing units in the adaptable assembly are processed in series, which is useful for sample processing applications.

The various embodiments described herein with regard to the hybrid, disposable and scalable SPTFF systems of the invention are equally applicable to the adaptable modular systems of the invention.

Also disclosed is a method of altering the processing capacity of an adaptable modular SPTFF assembly of the invention, comprising adding or removing at least one processing unit to or from the assembly;
replacing at least one TFF cassette in one or more processing units in the assembly with a TFF cassette having a different filtration membrane area; adding or removing at least one TFF cassette to or from one or more processing units in the assembly; or
a combination thereof.


*Single-Pass Tangential Flow Filtration (SPTFF) Processes and Methods*

The disclosure further relates to processes and methods for increasing product recovery from SPTFF systems and processes and methods for cleaning SPTFF assemblies. SPTFF systems that are particularly suitable for use in the processes and methods described herein include, for example, the partial recirculation TFF, hybrid, disposable and scalable SPTFF systems/assemblies that are described herein above. Other TFF systems capable of being used in the processes and methods described herein are well known in the art to which the present invention pertains and include, but are not limited to, those described in U.S. Patent No. 5,147,542 and commercially available TFF systems from EMD Millipore Corporation (Billerica, MA) including, for example, Labscale™ TFF Systems, Cogent M1 TFF Systems, Cogent μScale TFF Systems, Flexware Assemblies for TFF, ProFlux TFF Systems, and Prostak TFF Systems. Other commercially available TFF Systems that can be operated in single pass mode include, e.g., Cadence™ Single Pass TFF Systems and Allegro™ TFF Systems (Pall Corporation, Port Washington, NY).


*Processes for Increasing Product Recovery from SPTFF Systems*

General methods for recovering product (e.g., target proteins) from batch TFF systems typically use an initial recirculation through the system to depolarize proteins adsorbed to the filtration membranes and increased their recovery during a subsequent air blow-down or buffer flush of the cassette feed channel. For SPTFF assemblies, however, a recirculation step cannot be employed to depolarize proteins adsorbed to the filtration membranes without introducing a recirculation loop and taking away the simplicity and ease-of-use benefits of SPTFF.

The present disclosure contemplates processes for recovering proteins from the surface of a filtration membrane in a single-pass tangential flow filtration (SPTFF) assembly using a static hold recovery step. As used herein, a "static hold recovery step" refers to a step during which the pressurized flow of liquid through the SPTFF system is stopped to allow permeate in the system to flow backward through the filtration membranes by osmosis. The backward flow of permeate through the membranes pushes proteins off the membrane surfaces and into the feed channel. The proteins removed from the membranes can subsequently be displaced from the feed channel, e.g., using a standard air blow-down or buffer flush.

Accordingly, the disclosure relates to a process for recovering proteins from the surface of a filtration membrane in a single-pass tangential flow filtration (SPTFF) assembly. The protein recovery process comprises the steps of introducing a liquid feed comprising proteins into a feed channel in a SPTFF assembly; passing the liquid feed across a filtration membrane along the feed channel, thereby separating the liquid feed into a retentate and a permeate; stopping the flow of the liquid feed across the membrane for a period of time sufficient to allow permeate to diffuse backwards through the membrane by osmosis, thereby displacing proteins from the surface of the membrane back into the feed channel; and recovering the displaced proteins from the feed channel.

The flow of liquid is stopped during the static recovery step for a period of time that is sufficient to allow permeate to remove a desired amount of target protein from the membrane surfaces, typically at least about 5 to about 15 minutes. A person of ordinary skill in the art to which the invention pertains can readily determine a suitable duration for the static recovery step.

Methods of recovering proteins from the feed channel of a SPTFF assembly are known in the art and include, for example, flushing the feed channel with a liquid (e.g., water, buffer) or dispensing pressurized air through the feed channel, also known as an "air blow-down."

The permeate in the assembly can be liquid permeate or dry permeate (e.g., a dry air or vacuum permeate). When the permeate is a dry permeate, the process can further include the step of introducing a liquid (e.g., water, buffer) to the dry permeate to facilitate diffusion of the permeate through the filtration membranes.

*Methods of Cleaning SPTFF Systems*

Routine cleaning of TFF assemblies is required to prevent batch-to-batch carryover and restore the membrane for batch-to-batch process consistency. Batch systems recirculate cleaning agents through the system to ensure adequate cleaning. However, recirculation of cleaning agents cannot be employed for the SPTFF flow configuration without introducing a recirculation loop and taking away the simplicity and ease-of-use benefits of SPTFF. Accordingly, there is a need for improved methods of cleaning SPTFF assemblies that restore SPTFF assemblies to desired levels of cleanliness in the absence of recirculation of cleaning agents.

The present disclosure contemplates methods of cleaning SPTFF assemblies that include a static cleaning step. As used herein, "static cleaning step" refers to a step during which the pressurized flow of liquid through the SPTFF system is stopped to allow cleaning agent (e.g., NaOH) in the system to diffuse by osmosis throughout the TFF cassettes and the filtration membranes therein. As described herein, a cleaning method employing a static cleaning step has been used successfully to clean an SPTFF assembly after intravenous immunoglobulin (IVIG).

The static cleaning step has a duration (e.g., typically at least about 30 to about 60 minutes) that is sufficient to allow the cleaning agent to eliminate contaminants in the system (e.g., by hydrolysis). A person of ordinary skill in the art to which the invention pertains can readily determine a suitable duration for the static cleaning step.

The disclosure provides a process for cleaning a TFF assembly (e.g., a SPTFF assembly, a batch TFF assembly), comprising the steps of flushing a liquid that lacks cleaning agent (e.g., water, buffer) through a feed channel in the TFF assembly for a period of time sufficient to allow the liquid to displace product from the surfaces of filtration membranes in the assembly and out of the feed channel; subsequently flushing the feed channel in the assembly with a cleaning solution comprising a cleaning agent, thereby removing the displaced product from the feed channel; stopping the flow of liquid through the assembly for a period of time sufficient to allow the cleaning agent to reach the internal surfaces of the assembly and diffuse into fouling deposits on the filtration membrane, thereby dissolving the fouling deposits; and flushing the assembly with a liquid that lacks the cleaning agent to remove residual cleaning agent from the assembly.

Exemplary methods for flushing a SPTFF assembly with a liquid (e.g., buffer, water, cleaning solution) are known in the art.

Persons of ordinary skill in this art can readily select an appropriate cleaning agent(s) for removing the particular type of contaminant(s) or foulant(s) (e.g., adsorbed protein, cell debris, lipids, polysaccharides, organic colloids, mineral deposits, metal complexes) that are sought to be removed from the membranes in the SPTFF system. Exemplary cleaning agents for SPTFF systems include, but are not limited to, NaOH, Tergazyme®, NaOCl, Triton® X-100, $H_3PO_4$,

SDS, citric acid, HNO$_3$, Tween®-80, urea, and HNO$_3$H$_3$PO$_4$, as well as various combinations thereof. Preferably, the cleaning agent is NaOH (e.g., about 0.5 M NaOH).

In some arrangements, the method also comprises the step of testing the assembly to determine whether the surface of the filtration membranes has been restored to a desired level of cleanliness. Methods of testing SPTFF assemblies for cleanliness are known in the art and include, for example, measuring the normalized water permeability (NWP) of TFF cassettes and filter membranes. Techniques for assessing NWP are well known in the art. Alternatively and/or in addition, the cleanliness of a SPTFF assembly can be tested after cleaning by flushing the system with a solution (e.g., buffer, water) that lacks cleaning agent and assaying for residual carry-over protein.

*Partial Recirculation TFF Methods (Not according to the Invention)*

The processes and methods described herein, can in some arrangements, be performed using partial recirculation TFF. Partial recirculation TFF relates to a method of filtering a liquid feed, comprising passing a liquid feed through a tangential flow filtration (TFF) system, recovering permeate and a portion of the retentate from the system in separate containers without recirculation through the TFF system, and recirculating the remainder of the retentate through the TFF system at least once. Recirculating all or a portion of the retentate during start up provides a method by which to ensure that the system has reached equilibrium and the retentate has achieved the desired concentration prior to collecting it into the product vessel. It also provides a convenient way to respond to system upsets during processing to provide a more robust process. The fraction of retentate that is recirculated can be adjusted via modulation of the pump or control valve as a way to tune the system in order to assure consistent retentate concentration and/or consistent retentate flow rate to the product collection vessel every run even if feedstock protein concentration, new membrane permeability, membrane fouling, membrane permeability, or membrane mass transfer or pressure drop varies from batch to batch. This strategy has particular benefits in the context of continuous processing where the success of subsequent operations rely on the output of a previous operation. Recirculation of retentate can improve cleaning effectiveness through increased cross flow velocity and reduce cleaning solution through recirculation. The TFF systems described above for use in the SPTFF methods of the disclosure typically are also useful for the TFF methods involving partial recirculation of retentate that are described herein. The TFF systems employed in the TFF methods involving recirculation additionally include at least one pump or control valve for recirculating retentate through all or part of the system and at least one conduit for recirculating (e.g., carrying) retentate.

Typically, in a TFF method involving partial recirculation of retentate, at least about 50% of the retentate is collected after a single pass, while the remainder of the retentate is recirculated. Preferably, about 10% or less (e.g., about 0.5%, about 1%, about 2%, about 5%, about 10%) of the retentate is recirculated after the first pass through the TFF system.

The amount of retentate that is recirculated can be controlled using, for example, a pump or a valve. A flow meter can be used to provide a process value for the pump or valve to control the amount of retentate that is recirculated. Thus, in some arrangements, the TFF systems described herein for use in the partial recirculation TFF methods can further comprise a valve or pump and/or a flow meter for controlling recirculation of retentate. Preferably, the valve or pump and/or flow meter is positioned on the retentate outlet or flow line carrying retentate out of the system to the retentate receptacle.

The retentate that is being recirculated can be returned to any upstream location in or before the TFF system. In one arrangement, the retentate is recirculated to the feed tank. In another arrangement, the retentate is recirculated to the feed line near the feed pump before the feed inlet on the TFF system.

*TFF Diafiltration Methods (Not according to the Invention)*

In some arrangements, the processes and methods described herein further comprise performing diafiltration (e.g., to remove or lower the concentration of salts or solvents in the liquid feed, or to accomplish buffer exchange). In a preferred arrangement, the diafiltration is performed by concentrating the liquid feed (e.g., by TFF) to reduce the diafiltration volume and then restoring the feed to its starting volume by adding diafiltration solution, a process which is known in the art as discontinuous, or batch, diafiltration. In another arrangement, the diafiltration is performed by adding the diafiltrate solution to retentate to increase the diafiltration volume followed by concentrating the sample to restore it to its original volume. In yet another arrangement, the diafiltration is performed by adding the diafiltration solution to unfiltered feed at the same rate that permeate is removed from the TFF system, a process which is known on the art as continuous, or constant-volume, diafiltration. Suitable diafiltration solutions are well known and include, for example, water and various aqueous buffer solutions.

To perform diafiltration, the TFF system employed can include a reservoir or container for diafiltration solution and one or more conduits for carrying diafiltration solution from the diafiltration solution container to the liquid feed tank.

To avoid extremes of concentration and in-line dilution as part of the diafiltration process (e.g.>90%), it is preferred to inject the diafiltrate into multiple sections of the filtration assembly to restore the flow in the retentate section to the same flow as in the initial feed. This requires matching the rate of diafiltrate buffer addition with the rate of permeate removal. A preferred method is to use a single pump with multiple pump heads containing the diafiltrate addition and permeate removal flow lines (e.g. Peristaltic pump from Ismatec (Glattbrugg, Switzerland). Each pump head will have closely-matched pumping rates so this process will be balanced and maintain efficient buffer exchange. It is recommended to match flows for each of the multiple sections by using pumps containing up to 24 channels.

*Example 1: A Hybrid SPTFF System For Operation of Cassettes in Parallel and/or in Series*

A SPTFF model was developed to evaluate and compare conversion, permeate flow rate, and pressure drop for three cassettes staged in series and three cassettes staged in parallel. The model was based on the Pellicon® device format with 30kD Ultracel membrane and C-screen (EMD Millipore). Experimental testing has indicated that feed flows of 0.25-0.5LMM are typically required to achieve the target conversion or concentration. SPTFF flushing steps should be performed at higher flow rates to reduce time. Pumps used for large scale processing commonly have flow ranges of 6-10X. Given these considerations, a feed flow rate for flushing steps of 1LMM was selected for this model. Each cassette was divided into 28 elements (~0.5cm each). The properties of the fluid and cassette were assumed to be constant within each element. The output of element n was used at the input to element n+1. The lower limit of the model was defined as an outlet pressure 0psig. The upper limit of the model was defined as 100% conversion (i.e. retentate flow rate = 0). Additional model inputs are defined below:

Membrane Permeability: about 145 LMH/bar (10 LMH/psi)
Viscosity = 1cPa

$$\text{Pressure Drop per element} = (1.56*Q + 0.255*Q^2)/28$$

Note: Pressure drop based on internal Millipore experimental data.
Feed Flow Rate = 1LMM

The permeate flush volume specification of 70L/m2 for Pellicon® 2 devices (EMD Millipore) was used in this example. The total flush times were calculated for total conversions of 70%, 80%, 90%, and 100%. For the serial example, flush time was calculated based on the permeate flow rate of the third stage. The estimated permeate flow rates are shown in Tables 1 and 2. The comparison of estimated flush times is shown in Table 3.

Table 1: Expected Flushing Flow Rates For Cassettes Operated in Parallel

| Total Conversion (%) | Inlet Pressure Bar (psig) | Outlet Pressure Bar (psig) | Segment 1 Permeate Flux (LMH)[1] | Retentate Flux (LMH)[1] |
|---|---|---|---|---|
| 70 | 0.336 (4.88) | 0.258 (3.74) | 42.00 | 18.00 |
| 80 | 0.376 (5.46) | 0.303 (4.40) | 48.00 | 12.00 |
| 90 | 0.416 (6.03) | 0.348 (5.05) | 54.00 | 6.00 |
| 100 | 0.455 (6.60) | 0.394 (5.71) | 60.00 | 0.00 |
| [1] Flux based on total system area (3X) | | | | |

Table 2: Expected Flushing Flow Rates For Cassettes Operated in Series

| Total Conversion (%) | Inlet Pressure Bar (psig) | Outlet Pressure Bar (psig) | Segment 1 Permeate Flux (LMH)[1] | Segment 2 Permeate Flux (LMH)[1] | Segment 3 Permeate Flux (LMH)[1] | Retentate Flux (LMH)[2] |
|---|---|---|---|---|---|---|
| 70 | 0.711 (10.30) | 0.042 (0.61) | 74.49 | 36.61 | 14.90 | 18.00 |
| 80 | 0.735 (10.66) | 0.119 (1.72) | 78.36 | 42.05 | 23.58 | 12.00 |

(continued)

| Total Conversion (%) | Inlet Pressure Bar (psig) | Outlet Pressure Bar (psig) | Segment 1 Permeate Flux (LMH)[1] | Segment 2 Permeate Flux (LMH)[1] | Segment 3 Permeate Flux (LMH)[1] | Retentate Flux (LMH)[2] |
|---|---|---|---|---|---|---|
| 90 | 0.760 (11.03) | 0.194 (2.81) | 82.28 | 47.54 | 32.19 | 6.00 |
| 100 | 0.789 (11.44) | 0.276 (4.00) | 86.70 | 53.70 | 39.61 | 0.00 |

[1] Flux based on area of individual stage (1X)
[2] Flux based on total system area (3X)

Table 3: Estimated Flush Times and Volumes for Serial and Parallel Cassette Processing

| Total Conversion (%) | Parallel Operation | | Serial Operation | |
|---|---|---|---|---|
| | Estimated Flush Time (hr) | Total Estimated Flush Volume (L/m$^2$) | Estimated Flush Time (hr) | Total Estimated Flush Volume (L/m$^2$) |
| 70 | 1.67 | 100.00 | 4.70 | 281.82 |
| 80 | 1.46 | 87.50 | 2.97 | 178.09 |
| 90 | 1.3 | 77.78 | 2.17 | 130.48 |
| 100 | 1.17 | 70.00 | 1.77 | 106.05 |

Although serial processing shows an overall improvement in conversion with proteins, it requires additional time and volume to flush the cassettes compared to parallel processing. Additional process time and volumes increases the overall cost of the unit operation. The above example is specific to initial pre-use preservative flushing, but the challenges associated with flushing cassettes staged in series will be consistent for all flushing steps. It is especially critical to ensure proper flushing when cleaning to ensure adequate exposure to cleaning solution. Since conversion and expected protein concentration will be highest in the final processing unit, the final processing unit is typically the most challenging to effectively clean. The final processing unit is also the most difficult to flush. It is critical to ensure proper flushing of this final processing unit during cleaning.

Large scale SPTFF installations with cassettes processed in series are expected to have a common permeate line. The permeate from all processing units feed a common manifold preventing operators from measuring the flow rates for individual units. When measuring normalized water permeability (NWP), the TMP and average cross flow rate will decrease for later units which can hinder water permeability measurements and prevent proper assessment of cleaning effectiveness. If cleaning solution is not adequately flushed through the third unit causing ineffective cleaning, it may be difficult to determine.

*Example 2: Effectiveness of a Cleaning Method for TFF Cassettes Employing Parallel Flushing and a Static Soak Step*

A study was carried out to evaluate the effectiveness of cleaning TFF cassettes by employing a static soak step with 0.5N NaOH over 20 SPTFF re-uses of the cassettes. Pellicon® 3 C-screen mini cassettes with 30 KD Ultracel membranes were used for this study. The TFF cassettes were incorporated into a hybrid SPTFF system of the invention that was capable of being operated in series or in parallel. The cassettes were cleaned by flushing in parallel and soaking with 0.5N Sodium Hydroxide (NaOH) for 20 cycles. One additional cycle was carried out to flush the cassettes in series to simulate a SPTFF set-up where cleaning in parallel was not feasible. This was done to determine if there is any difference in the cassettes' performance recovery between flushing the cassettes in parallel and series. IgG (SeraCare Life Sciences, Inc., Milford, MA) was used as model feed. A full 4-hour SPTFF process was run before each cleaning cycle.

Twenty (20) cycles over a 4-hour duration were conducted at 75ml/min (0.2LMM) at a protein concentration range of 120-150g/L to meet process scale conditions. Retentate pressure was controlled at 0.69-1.03 bar (10-15psi) and process flux was measured to check for process reproducibility. The cleaning cycle consisted of flushing 0.5N NaOH in parallel at a feed flow rate of 450ml/min (1.4LMM) for 15 minutes followed by soaking 0.5N NaOH for 45 minutes. Following each cleaning cycle, purified flush water Total Organic Carbon (TOC) was tracked in permeate line and Normalized Buffer Permeability (NBP) was measured.

The effectiveness of the cleaning cycles was evaluated and compared using the following criteria:

- Process Normalized Buffer Permeability (NBP) - measured in parallel flow configuration
- Process flux, conversion and pressure drop;
- Product retention; and
- Residual Total Organic Count (TOC) in Post Cleaning Water Flush.

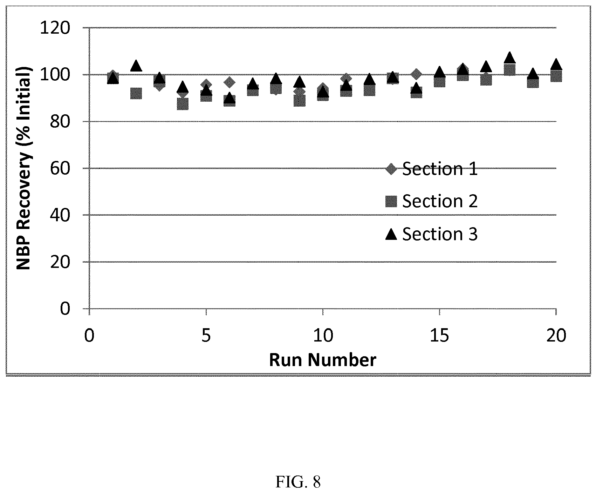

The recovery of NBP is shown in FIG. 8. The NBP of the individual sections is plotted to better show cleaning effectiveness. The test results showed that this static soaking cleaning method is effective to restore the membrane performance with more than 90% NBP recovery after every cleaning cycle.

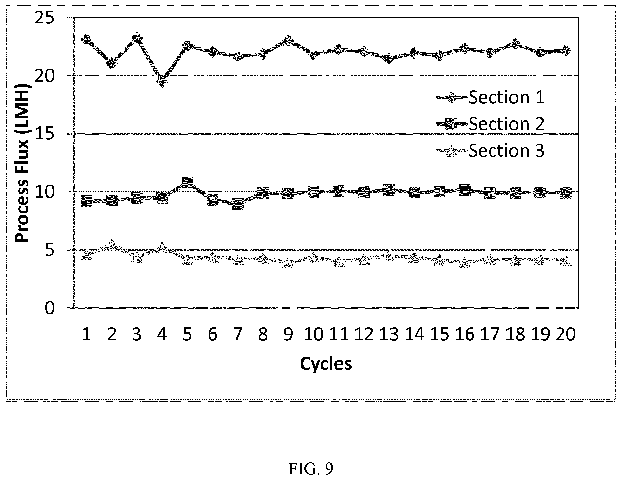

The process flux after re-use with static cleaning is shown in FIG. 9. The process flux of the individual sections is plotted to better show cleaning effectiveness.

The cassettes demonstrated reproducible process flux for all three stages in the 20 cycles of concentration.

Additionally, the process maintained >99.8% protein retention throughout the cleaning evaluation. All flush TOC samples after the cleaning cycles showed less than 1ppm TOC.

While this invention has been particularly shown and described with references to example embodiments thereof, it will be understood by those skilled in the art that various changes in form and details may be made therein without departing from the scope of the invention encompassed by the appended claims.

## Claims

1. A single pass tangential flow filtration (SPTFF) assembly, comprising:

   a plurality of processing units (1, 2, 3) that are fluidly connected, wherein each processing unit comprises a cassette holder containing at least one TFF cassette, and wherein the processing units in the assembly are configured for processing in parallel and/or in series; a first channel (4) connected to a feed inlet (5) extending through the plurality of processing units;
   a second channel (6) connected to a retentate outlet (7) extending through the plurality of processing units;
   a permeate channel (8) extending through the plurality of processing units; and
   one or more valves (9, 10), wherein each valve is located on either the first channel or the second channel between two adjacent processing units, and
   wherein the valves alternate between the first channel and second channel for consecutive pairs of adjacent processing units;

   wherein the plurality of processing units are fluidly connected in parallel when the one or more valves are open, or in series when the one or more valves are closed; and wherein the one or more valves are built into the cassette holders.

2. The SPTFF assembly of Claim 1, wherein:

   the assembly further comprises 1) a spool piece (11) on the second channel between each pair of adjacent processing units having a valve on the first channel; or 2) a spool piece (11) on the first channel between each pair of adjacent processing units having a valve on the second channel;
   and/or
   the plurality of processing units are stacked vertically; 2) the assembly contains two processing units; 3) the assembly contains two processing units; or 4) the assembly contains three processing units; or 4) the assembly contains more than three processing units;
   and/or
   wherein the one or more valves are sanitary valves.

3. The SPTFF assembly of Claim 1, wherein the SPTFF assembly comprises disposable components, the disposable components including:

   a disposable feed line in fluid connection with the first channel;
   the at least one TFF cassette being a disposable TFF cassette;
   the holder for the at least one TFF cassette being a disposable holder;
   a disposable retentate line in fluid connection with the second channel; and

a disposable retentate valve,
wherein fluid contact surfaces on the disposable components are contained in a sealed disposable container that comprises tubing for making aseptic connections to feed and product vessels.

4. The SPTFF assembly of Claim 3, wherein:

1) the one or more disposable components are made from plastic; or 2) the one or more disposable components are replaceable;

and/or
the assembly comprises a plurality of TFF cassettes and holders, wherein the holders are stacked to produce a stacked assembly;
and/or
the assembly comprises a plurality of disposable holders, wherein the holders are fluidly connected in parallel;
and/or
wherein the retentate line further comprises a T-line for in-line buffer addition; and/or
the assembly further comprises at least one of: 1) a disposable pressure sensor;

2) a disposable diaphragm for a pressure sensor; 3) one or more retentate end plates for a stacked assembly, wherein the retentate end plates are disposable; and 4) a disposable bioprocess container for feed in a mechanical support that allows pressurization.

5. A process for recovering proteins from the surface of a filtration membrane in a single pass tangential flow filtration (SPTFF) assembly, comprising:

a) introducing a liquid feed comprising proteins into a feed channel in a SPTFF assembly of any one of Claims 1-4;
b) passing the liquid feed across a filtration membrane along the feed channel, thereby separating the liquid feed into a retentate and a permeate;
c) stopping the flow of the liquid feed across the membrane for a period of time sufficient to allow permeate to diffuse backwards through the membrane by osmosis, thereby displacing proteins from the surface of the membrane back into the feed channel; and
d) recovering the displaced proteins from the feed channel.

6. The process of Claim 5, wherein:
the proteins are recovered from the feed channel by flushing the feed channel with a liquid; or the proteins are recovered from the feed channel by dispensing pressurized air through the feed channel.

7. A process for cleaning a single-pass tangential flow filtration (SPTFF) assembly, comprising:

a) flushing a liquid that lacks cleaning agent (e.g., water, buffer) through a feed channel in a SPTFF assembly of any one of Claims 1-4 for a period of time sufficient to allow the liquid to displace product from the surfaces of filtration membranes in the assembly to the feed channel;
b) flushing the feed channel in the assembly with a cleaning solution comprising a cleaning agent, thereby removing the displaced product form the feed channel;
c) stopping the flow of liquid through the assembly for a period of time sufficient to allow the cleaning agent to reach the internal surfaces of the assembly and diffuse into fouling deposits on the filtration membrane, thereby dissolving the fouling deposits; and
d) flushing the assembly with a liquid that lacks the cleaning agent to remove residual cleaning agent from the assembly.

8. The process of Claim 7, wherein:

the process further comprises testing the assembly to determine whether the surface of the filtration membrane has been restored to a desired level of cleanliness;
and/or
the cleaning agent is NaOH.

9. A method of installing a disposable single-pass tangential flow filtration (SPTFF) assembly, comprising:

   a) establishing a first aseptic connection between a feed line of a disposable SPTFF assembly of Claim 3 and a feed container; and
   b) establishing a second aseptic connection between a retentate line of the disposable SPTFF assembly and a product container.

10. A method of increasing the processing capacity of a single pass tangential flow filtration (SPTFF) assembly, comprising:

   a) increasing the filtration membrane area in a SPTFF assembly of any one of Claims 1-4; and
   b) increasing the volumetric flow of feed solution that is being delivered to the SPTFF assembly to a level that is proportional to the increase in filtration membrane area;
   wherein the residence time of the feed solution and the length of the flowpath in the TFF assembly are maintained.

11. The method of Claim 10, wherein the filtration membrane area in the assembly is increased by 1) replacing at least one TFF cassette in the assembly with a larger TFF cassette; or 2) adding at least one TFF cassette to the assembly.

**Patentansprüche**

1. Tangentialflussfiltrationsanordnung mit einfachem Durchgang (SPTFF-Anordnung) umfassend:

   eine Vielzahl von Verarbeitungseinheiten (1, 2, 3), die fluidisch verbunden sind, wobei jede Verarbeitungseinheit einen Kassettenhalter umfasst, der mindestens eine TFF-Kassette enthält, und wobei die Verarbeitungseinheiten in der Anordnung dazu konfiguriert sind, parallel und/oder in Reihe zu verarbeiten; einen ersten Kanal (4), der mit einem Zuführeinlass (5) verbunden ist, der sich durch die Vielzahl von Verarbeitungseinheiten erstreckt; einen zweiten Kanal (6), der mit einem Retentatauslass (7) verbunden ist, der sich durch die Vielzahl von Verarbeitungseinheiten erstreckt;
   einen Permeatkanal (8), der sich durch die Vielzahl von Verarbeitungseinheiten erstreckt; und ein oder mehrere Ventile (9, 10), wobei sich jedes Ventil entweder am ersten Kanal oder am zweiten Kanal zwischen zwei benachbarten Verarbeitungseinheiten befindet, und wobei die Ventile zwischen dem ersten Kanal und dem zweiten Kanal abwechseln für aufeinanderfolgende Paare von benachbarten Verarbeitungseinheiten;

   wobei die Vielzahl von Verarbeitungseinheiten fluidisch parallelgeschaltet sind, wenn das eine oder die mehreren Ventile geöffnet sind, oder in Reihe, wenn das eine oder die mehreren Ventile geschlossen sind; und wobei das eine oder die mehreren Ventile in die Kassettenhalter eingebaut sind.

2. SPTFF-Anordnung nach Anspruch 1, wobei:

   die Anordnung ferner 1) einen Spulenteil (11) auf dem zweiten Kanal zwischen jedem Paar von benachbarten Verarbeitungseinheiten, die ein Ventil am ersten Kanal haben; oder 2) ein Spulenteil (11) auf dem ersten Kanal zwischen jedem Paar von benachbarten Verarbeitungseinheiten, die ein Ventil am zweiten Kanal haben, umfasst;
   und/oder
   die Vielzahl von Verarbeitungseinheiten vertikal gestapelt sind; 2) die Anordnung zwei Verarbeitungseinheiten enthält; 3) die Anordnung zwei Verarbeitungseinheiten enthält; oder 4) die Anordnung drei Verarbeitungseinheiten enthält; oder 4) die Anordnung mehr als drei Verarbeitungseinheiten enthält;
   und/oder
   wobei das eine oder die mehreren Ventile Sanitärventile sind.

3. SPTFF-Anordnung nach Anspruch 1, wobei die SPTFF-Anordnung Einwegbestandteile umfasst, wobei die Einwegbestandteile Folgendes beinhalten:

   eine Einwegzuführleitung, die in fluidischer Verbindung mit dem ersten Kanal ist;
   die mindestens eine TFF-Kassette, die eine Einwegkassette ist;
   den Halter für die mindestens eine TFF-Kassette, der ein Einweghalter ist;
   eine Einwegretentatleitung, die in fluidischer Verbindung mit dem zweiten Kanal ist; und ein Einwegretentatventil,

wobei fluidische Kontaktoberflächen auf den Einwegbestandteilen in einem versiegelten Einwegbehälter enthalten sind, der Leitungen zum Herstellen aseptischer Verbindungen zu Zufuhr- und Produktbehältern umfasst.

4. SPTFF-Anordnung nach Anspruch 3, wobei:

1) das eine oder die mehreren Einwegbestandteile aus Plastik gemacht sind; oder 2) das eine oder die mehreren Einwegbestandteile austauschbar sind;

und/oder
die Anordnung eine Vielzahl von TFF-Kassetten und Haltern umfasst, wobei die Halter gestapelt sind, um eine gestapelte Anordnung zu produzieren;
und/oder
die Anordnung eine Vielzahl von Einweghaltern umfasst, wobei die Halter fluidisch parallelgeschaltet sind;
und/oder
wobei die Retentatleitung ferner eine T-Leitung umfasst für lineare Pufferergänzung;
und/oder
die Anordnung ferner mindestens eins der Folgenden umfasst: 1) einen Einwegdrucksensor; 2) eine Einwegmembran für einen Drucksensor; 3) eine oder mehrere Retentatendplatten für eine gestapelte Anordnung, wobei die Retentatendplatten wegwerfbar sind; und 4) einen Einwegbioprozessbehälter zur Zufuhr in einer mechanischen Unterstützung, die Druckbeaufschlagung erlaubt.

5. Prozess zum Wiederherstellen von Proteinen von der Oberfläche einer Filtrationsmembran in einer Tangentialflussfiltrationsanordnung mit einfachem Durchgang (SPTFF-Anordnung) umfassend:

a) Einspeisen einer Flüssigkeitszufuhr, die Proteine umfasst, in einen Zuführungskanal in einer SPTFF-Anordnung nach einem der Ansprüche 1-4;
b) Durchlaufen der Flüssigkeitszufuhr durch eine Filtrationsmembran entlang des Zuführungskanals, wodurch die Flüssigkeitszufuhr in ein Retentat und in ein Permeat geteilt wird;
c) Stoppen des Flusses der Flüssigkeitszufuhr durch die Membran für einen Zeitraum, der ausreichend ist, um dem Permeat zu erlauben, sich rückwärts durch die Membran durch Osmose zu verbreiten, wodurch Proteine von der Oberfläche der Membran zurück in den Zuführungskanal verschoben werden; und
d) Wiederherstellen der verschobenen Proteine aus dem Zuführungskanal.

6. Prozess nach Anspruch 5, wobei:
die Proteine wiederhergestellt werden aus dem Zuführungskanal durch Spülen des Zuführungskanals mit einer Flüssigkeit; oder die Proteine wiederhergestellt werden aus dem Zuführungskanal durch Ausgeben von unter Druck stehender Luft durch den Zuführungskanal.

7. Prozess zum Reinigen einer Tangentialflussfiltrationsanordnung mit einfachem Durchgang (SPTFF-Anordnung) umfassend:

a) Spülen einer Flüssigkeit, der Reinigungsmittel fehlt (z.B. Wasser, Puffer) durch einen Zuführungskanal in einer SPTFF-Anordnung nach einem der Ansprüche 1-4 für einen Zeitraum, der ausreichend ist, um der Flüssigkeit zu erlauben, Produkt von der Oberfläche der Filtrationsmembranen in der Anordnung zum Zuführungskanal zu verschieben;
b) Spülen des Zuführungskanals in der Anordnung mit einer Reinigungslösung, die ein Reinigungsmittel umfasst, wodurch das verschobene Produkt aus dem Zuführungskanal entfernt wird;
c) Stoppen des Flusses der Flüssigkeit durch die Anordnung für einen Zeitraum, der ausreichen ist, um dem Reinigungsmittel zu erlauben, die inneren Oberflächen der Anordnung zu erreichen und sich in Schmutzniederschlägen auf der Filtrationsmembran zu verbreiten, wodurch die Schmutzniederschläge aufgelöst werden; und
d) Spülen der Anordnung mit einer Flüssigkeit, der Reinigungsmittel fehlt, um übriges Reinigungsmittel aus der Anordnung zu entfernen.

8. Prozess nach Anspruch 7, wobei:

der Prozess ferner das Testen der Anordnung umfasst, um zu bestimmen, ob die Oberfläche der Filtrationsmembran auf ein gewünschtes Maß an Sauberkeit wiederhergestellt wurde;

und/oder
das Reinigungsmittel NaOH ist.

9. Verfahren zum Installieren einer Einweg-Tangentialflussfiltrationsanordnung mit einfachem Durchgang (SPTFF-Anordnung), umfassend:

a) Schaffen einer ersten aseptischen Verbindung zwischen einer Zuführungsleitung einer Einweg-SPTFF-Anordnung nach Anspruch 3 und eines Zufuhrbehälters; und
b) Schaffen einer zweiten aseptischen Verbindung zwischen einer Retentatleitung der Einweg-SPTFF-Anordnung und eines Produktbehälters.

10. Verfahren zum Steigern der Verarbeitungskapazität einer Tangentialflussfiltrationsanordnung mit einfachem Durchgang (SPTFF-Anordnung), umfassend:

a) Steigern des Filtrationsmembranbereichs in einer SPTFF-Anordnung nach einem der Ansprüche 1-4; und
b) Steigern des Volumendurchflusses von Zufuhrlösung, die zur SPTFF-Anordnung auf einem Niveau geliefert wird, das proportional zu der Steigerung des Filtrationsmembranbereichs ist;

wobei die Verweilzeit der Zufuhrlösung und die Länge des Durchflusswegs in der TFF-Anordnung aufrechterhalten werden.

11. Verfahren nach Anspruch 10, wobei der Filtrationsmembranbereich in der Anordnung gesteigert ist durch 1) Ersetzen von mindestens einer TFF-Kassette in der Anordnung mit einer größeren TFF-Kassette; oder 2) Ergänzen von mindestens einer TFF-Kassette in die Anordnung.

**Revendications**

1. Ensemble de filtration à flux tangentiel à passage unique (SPTFF), comprenant :

une pluralité d'unités de traitement (1, 2, 3) qui sont connectées de manière fluide, dans lequel chaque unité de traitement comprend un porte-cassette contenant au moins une cassette TFF, et dans lequel les unités de traitement de l'ensemble sont configurées pour un traitement en parallèle et / ou en série ;
un premier canal (4) connecté à une entrée d'alimentation (5) s'étendant à travers la pluralité d'unités de traitement ;
un deuxième canal (6) connecté à une sortie de rétentat (7) s'étendant à travers la pluralité d'unités de traitement ;
un canal de perméat (8) s'étendant à travers la pluralité d'unités de traitement ; et
une ou plusieurs vannes (9, 10), dans lequel chaque vanne est située sur le premier canal ou le deuxième canal entre deux unités de traitement adjacentes, et dans lequel les vannes alternent entre le premier canal et le deuxième canal pour des paires consécutives d'unités de traitement adjacentes ;
dans lequel la pluralité d'unités de traitement sont connectées de manière fluide en parallèle lorsque la ou les vannes sont ouvertes, ou en série lorsque la ou les vannes sont fermées ; et
dans lequel la ou les vannes sont intégrées dans les supports de cassette.

2. Ensemble SPTFF selon la revendication 1, dans lequel :

l'ensemble comprend en outre 1) une pièce de bobine (11) sur le deuxième canal entre chaque paire d'unités de traitement adjacentes ayant une vanne sur le premier canal ; ou 2) une pièce de bobine (11) sur le premier canal entre chaque paire d'unités de traitement adjacentes ayant une vanne sur le deuxième canal ; et / ou
la pluralité d'unités de traitement sont empilées verticalement ; 2) l'ensemble contient deux unités de traitement ; 3) l'ensemble contient deux unités de traitement ; ou 4) l'ensemble contient trois unités de traitement ; ou 4) l'ensemble contient plus de trois unités de traitement; et / ou
dans lequel la ou les vannes sont des vannes sanitaires.

3. Ensemble SPTFF selon la revendication 1, dans lequel l'ensemble SPTFF comprend des composants jetables, les composants jetables incluant :

une ligne d'alimentation jetable en connexion fluidique avec le premier canal ;

l'au moins une cassette TFF étant une cassette TFF jetable ;
le support pour l'au moins une cassette TFF étant un support jetable ;
une ligne de rétentat jetable en connexion fluidique avec le deuxième canal ; et
une vanne de rétentat jetable, dans laquelle les surfaces de contact de fluide sur les composants jetables sont contenues dans un récipient jetable scellé qui comprend un tube pour établir des connexions aseptiques avec les récipients d'alimentation et de produit.

4. Ensemble SPTFF selon la revendication 3, dans lequel :

1) le ou les composants jetables sont en plastique ; ou 2) le ou les composants jetables sont remplaçables ;
et / ou
l'ensemble comprend une pluralité de cassettes TFF et de supports, les supports étant empilés pour produire un ensemble empilé ;
et / ou
l'ensemble comprend une pluralité de supports jetables, les supports étant connectés fluidiquement en parallèle ;
et / ou
dans lequel la ligne de rétentat comprend en outre une ligne T pour l'addition de tampon en ligne ;
et / ou
l'ensemble comprend en outre au moins l'un parmi : 1) un capteur de pression jetable ; 2) un diaphragme jetable pour un capteur de pression ; 3) une ou plusieurs plaques d'extrémité de rétentat pour un ensemble empilé, les plaques d'extrémité de rétentat étant jetables ; et 4) un récipient de bioprocédés jetable pour l'alimentation dans un support mécanique qui permet la pressurisation.

5. Procédé pour récupérer des protéines de la surface d'une membrane de filtration dans un ensemble de filtration à flux tangentiel à passage unique (SPTFF), comprenant :

a) l'introduction d'une alimentation liquide comprenant des protéines dans un canal d'alimentation dans un ensemble SPTFF selon l'une quelconque des revendications 1 à 4 ;
b) le passage de l'alimentation liquide à travers une membrane de filtration le long du canal d'alimentation, séparant ainsi l'alimentation liquide en un rétentat et un perméat ;
c) l'arrêt de l'écoulement de l'alimentation liquide à travers la membrane pendant une période de temps suffisante pour permettre au perméat de se diffuser vers l'arrière à travers la membrane par osmose, déplaçant ainsi les protéines de la surface de la membrane vers le canal d'alimentation ; et
d) la récupération des protéines déplacées du canal d'alimentation.

6. Procédé selon la revendication 5, dans lequel :
les protéines sont récupérées du canal d'alimentation en rinçant le canal d'alimentation avec un liquide ; ou les protéines sont récupérées du canal d'alimentation en distribuant de l'air sous pression à travers le canal d'alimentation.

7. Procédé de nettoyage d'un ensemble de filtration à flux tangentiel à passage unique (SPTFF) comprenant :

a) le rinçage d'un liquide qui manque d'agent de nettoyage (par exemple, de l'eau, du tampon) à travers un canal d'alimentation dans un ensemble SPTFF selon l'une quelconque des revendications 1 à 4 pendant une période de temps suffisante pour permettre au liquide de déplacer le produit depuis les surfaces des membranes de filtration dans l'ensemble vers le canal d'alimentation ;
b) le rinçage du canal d'alimentation dans l'ensemble avec une solution de nettoyage comprenant un agent de nettoyage, éliminant ainsi le produit déplacé du canal d'alimentation ;
c) l'arrêt de l'écoulement de liquide à travers l'ensemble pendant une période de temps suffisante pour permettre à l'agent de nettoyage d'atteindre les surfaces internes de l'ensemble et de se diffuser dans les dépôts d'encrassement sur la membrane de filtration, dissolvant ainsi les dépôts d'encrassement ; et
d) le rinçage de l'ensemble avec un liquide sans agent de nettoyage pour éliminer l'agent de nettoyage résiduel de l'ensemble.

8. Procédé selon la revendication 7, dans lequel :

le procédé comprend en outre le test de l'ensemble pour déterminer si la surface de la membrane de filtration a été restaurée au niveau de propreté souhaité ;

et / ou
l'agent de nettoyage est NaOH.

9. Procédé d'installation d'un ensemble jetable de filtration à flux tangentielle à passage unique (SPTFF), comprenant :

a) l'établissement d'une première connexion aseptique entre une ligne d'alimentation d'un ensemble SPTFF jetable selon la revendication 3 et un récipient d'alimentation ; et
b) l'établissement d'une deuxième connexion aseptique entre une ligne de rétentat de l'ensemble SPTFF jetable et un récipient de produit.

10. Procédé pour augmenter la capacité de traitement d'un ensemble de filtration à flux tangentiel à passage unique (SPTFF), comprenant :

a) l'augmentation de la surface de membrane de filtration dans un ensemble SPTFF selon l'une quelconque des revendications 1 à 4 ; et
b) l'augmentation du débit volumétrique de la solution d'alimentation qui est délivrée à l'ensemble SPTFF à un niveau qui est proportionnel à l'augmentation de la surface de la membrane de filtration ;
dans lequel la durée de séjour de la solution d'alimentation et la longueur du trajet d'écoulement dans l'ensemble TFF sont maintenus.

11. Procédé selon la revendication 10, dans lequel la surface de la membrane de filtration dans l'ensemble est augmentée en 1) remplaçant au moins une cassette TFF dans l'ensemble par une cassette TFF plus grande ; ou 2) en ajouter au moins une cassette TFF à l'ensemble.

FIG. 1

FIG. 2

FIG. 3

FIG. 4

FIG. 5

FIG. 6

FIG. 7

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030111402 A
- WO 2007076496 A
- US 5147542 A

**Non-patent literature cited in the description**

- **VAN REIS et al.** Linear Scale Ultrafiltration. *Biotechnology and Bioengineering,* 1997, vol. 55 (5), 737-746